# EUROPEAN PATENT SPECIFICATION

(11) **EP 1 852 084 B1**
(45) Date of publication and mention of the grant of the patent: **09.02.2011**
(21) Application number: 07014489.4
(22) Date of filing: 23.06.2006
(51) Int. Cl.: A61B 19/02, A61L 2/26

(54) **Sterilization container for surgical instruments**
Sterilisationsbehälter für chirurgische Instrumente
Récipient de stérilisation pour des instruments chirurgicaux

(30) Priority: 27.06.2005 IT mi20051209
(43) Date of publication of application: 07.11.2007
(62) Divisional of application: 06765585.2
(73) Proprietor: Steritalia S.p.A., 06019 Umbertide (PG) (IT)
(72) Inventor: Staccini, Umbro, 06015 Pirantonio (PG) (IT); Anzanello, Elisabetta Maria, 06015 Pirantonio (PG) (IT)
(74) Representative: Marietti, Andrea

(56) References cited:
- DE-A1- 2 603 912
- DE-A1- 19 858 418
- GB-A- 945 190
- US-A- 4 551 311
- US-A- 4 772 418
- US-A- 5 019 345
- US-B1- 6 620 390

## Description

### Field Of the invention

The present invention relates to a container and method according to the preamble of the independent claims.

### Background Art

US4551311 refers to closable containers for holding items that are subjected to sterilization processes and for storing and transporting such items.

DE2603912 discloses a container for a set of sterilized surgical instruments.

US6620390 relates to a-sterilizing container for holding surgical instruments or other sterilizing material during sterilization and for maintaining the sterilized condition during storage and transportation

US5019345 discloses a sterilizing containers are used to accommodate clinical sterile material which must be subjected to a steam sterilization.

US4772418 discloses container comprising a top cover for placement on a base support. The top cover may be placed in a first position on the base support to allow steam or gas to enter within the container and a second position for providing a seal between the cover and base support.

GB945190 discloses a plastics container the interior of which may be sterilized, and which is provided with means for maintaining the container in the sterilised state

### Summary Of the invention

The invention is defined by the independent claims.

Further purposes are achieved by a method not according to the present invention for the arrangement of sterilized procedural sets of surgical instruments for service to the operating rooms with cyclical and traceable operations of sanitizing, maintenance, sterilization and movement. It permits to identify a set of surgical instruments for every type of surgery, to furnish a graphic reproduction for the recognition of the instruments, to arrange one or more sets of surgical instruments for every type of surgery, in which every set is ordered in special metal grill-racks prepared in a closed container, sterilized in autoclave and equipped with a cover for evacuation. The sterilized set is then protected by a plastic film on which is affixed a photographic reproduction of the instruments, identification labels and batch documentation for the trackability of the procedures. In this way, the closed, sterile and sealed containers can be stored ready for use in quantity and type commensurate to the workloads of the operating room for surgical activity both programmed and in emergency. After the use of said instruments, the same are picked up in the container, pre-treated inside this latter with disinfecting solutions, closed with a cover for evacuation and collected in special trolleys for transport to the sterilization facility for the execution of decontamination, washing, and thermo-chemical disinfection. The instruments are checked and subject to possible maintenance or substitution where necessary, repackaged into ordered sets in said special metal racks, closed in the container and subjected to sterilization in autoclave with consequent repetition of all the preceding steps.

Further characteristics and advantages of the present method will mostly become evident from the detailed description of a form of preferred - but not exclusive - execution of a method for arrangement of sterilized procedural set of surgical instruments for service to operating rooms with cyclical and traceable treatment of sanitizing, maintenance, sterilization and movement, as well as of a structure at least devoted to the carrying out of the principal steps of such method.

### Brief Description Of the Drawings

The attached figures refer to a particular embodiment of the method and of the structure comprising suitable environments and equipment for a preferred but not exclusive execution thereof.

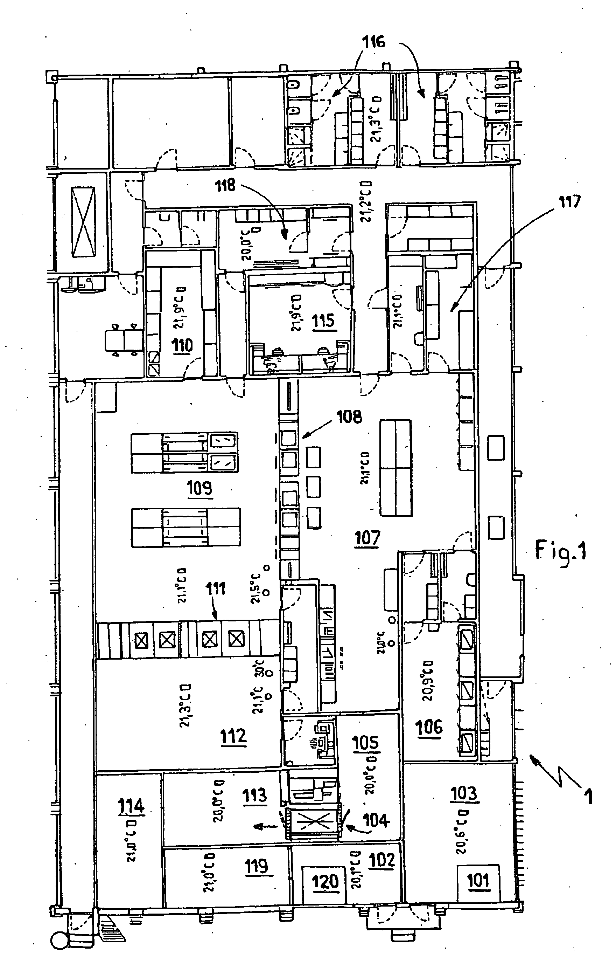

- Figure 1 is a plan view of the environment which is part of the patented structure in which the operations of sanitizing, disinfection, packaging and sterilization of surgical instruments for service in operating rooms are carried out;

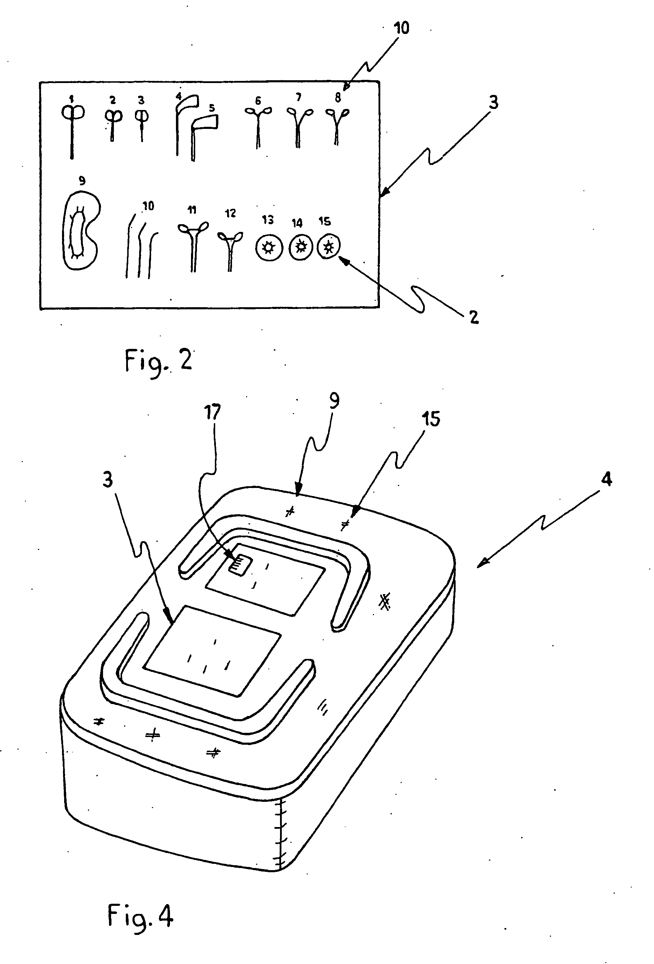

- Figure 2 is a graphic reproduction of a set of surgical instruments;

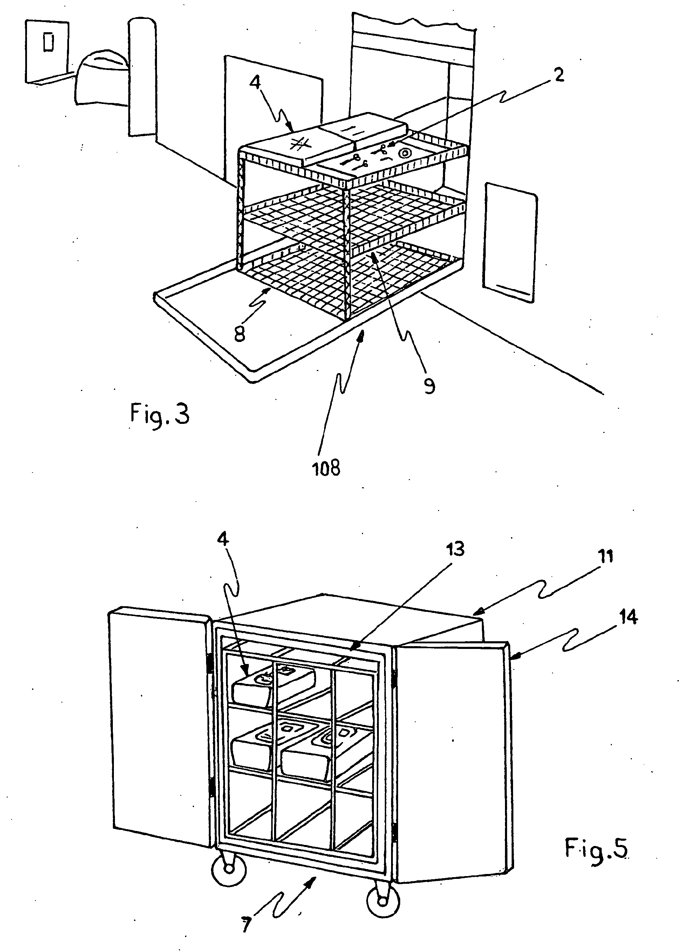

- Figure 3 is a perspective view of a surgical instrument washing machine
- Figure 4 is a perspective view of a container of surgical instruments, closed and comprising graphic means for the identification of the contents;
- Figure 5 is a perspective view of a collection trolley for closed containers;

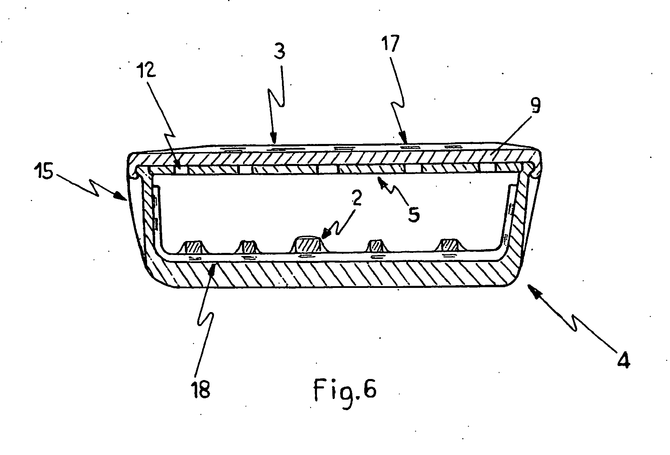

- Figure 6 is a view in section of the container of figure 4.

### Detailed Description Of A Particular Embodiment Of the invention

The method will now be described following the steps that are carried out inside suitable environments of the structure, making reference to the Figures described above.

With particular reference to such Figures, the numeral 1 indicates the whole set of environments in which are carried out the various steps of the operations of sanitizing, disinfection, packaging and sterilization of surgical instruments for service to operating rooms, said environments being preferably part of a service structure separated from the hospital or hospitals served.

The term "structure" used here and hereinafter shall refer to the entire set of environments for sanitizing, maintenance, disinfection, packaging and sterilization.

Furthermore, although a particular layout of the environments dedicated to the various steps of the operations of sanitizing, disinfection, packaging and sterilization of surgical instruments for service to operating rooms, is herein described, as will be clear to the skilled person, such a layout cannot be considered limiting to the present method; also not intended to be limiting to the method are the forms, the dimensions and the layout of the environments, the layout of the various apparatus set in the environments themselves and which serve for the operations of sanitizing, disinfection, packaging and sterilization of surgical instruments for service to operating rooms; also not limiting to the method is the type of equipment used for the execution of the said operations.

Step a) of the method which is the subject of the present invention relates to the identification of the set of surgical instruments 2 most suitable for the execution of each particular surgery and its graphic reproduction 3. Said identification is carried out, for instance, on the basis of the type of the surgery and usually in agreement with the surgeon who performs the surgery, with consequent choice of the quality, type and number of the surgical instruments 2 to be adopted. Once the set of surgical instruments 2 is identified, one proceeds to the choice of the layout of the instruments inside the container in which these are set aside, of the support racks 18 on which said instruments 2 are supported, and of the container 4 or of the series of containers, substantially parallelepiped in shape, most suited to receive said rack 18 or said racks and said surgical instruments 2.

The graphic reproduction 3 of the distribution of said surgical instruments 2 on every support rack 18 is produced, for instance, by a photographic device, with the additional insertion of identification numbers 10 of the relative position to every surgical tool 2.

Step b) of the method which is the subject of the present invention relates to the arrangement of one or more sets of surgical instruments 2 for every surgery and, where necessary, for every surgeon who will carry out the operation, selected on the basis defined in the aforesaid step a), the set being sterilized and orderly placed in a related container 4, which is closed with a sterilization cover 5 having passages 12 for the sterilization steam, with a second means of coverage 9 for closing the container 4 during evacuation, and with a plastic film 15 (for instance, a thermo-shrink plastic film), for airtight sealing of the closed container 4. Between said closed container 4 and said plastic film 15 is placed the graphic reproduction 3 of the set of surgical instruments contained therein. Together with the graphic reproduction 3 is preferably placed a further means of identification of the set of surgical instruments 2, such as, for instance, a bar code 17 and/or a further identification code such as a color code (here not shown) varying according to the discipline or to the surgery type of the set, rather than an alphanumeric denomination. Said means of sterilization cover 5 and evacuation cover 9 are fitted to the container 4 by, for instance, a restraining, interference or snap-lock means of fixture. Said surgical instruments 2 are positioned inside the container 4 supported on one or more support racks 18 suitably made for hosting a set of surgical instruments 2 for each surgery and for each surgeon.

Step c) of the method object of the present invention relates to the storage of the closed containers 4 in a suitable room, preferably, but not exclusively, in proximity of the operating room of the hospital in which said surgical instruments 2 are used for that particular surgery and for every surgeon destined to carry out the operation. The transport of said closed container 4 to the room of storage, preferably, but not exclusively, in proximity of the operating room of the hospital is effected with the aid of a means of transport of the trolley 7 type, comprising a first external hollow body 11 substantially parallelepiped in shape with airtight edges 13 and a second inside body comprising a series of rods connected such a way as to provide a shelf for supporting a plurality of said containers 4.

Said collection trolley 7 is previously sanitized and it is provided with doors 14 for airtight closure in correspondence to its edges.

Said step c) can be preceded, according to a preferred - but not exclusive - embodiment of this method, by an operation of transport in the hospital department by a vehicle suitably designed to receive at least one collection trolley 7, each trolley containing at least one closed container 4, to guarantee maximum stability during transport.

Step d) of the method object of the invention relates to the collection of the closed container 4 from the storeroom of the operating room, on the basis of a programmed or emergency surgery, transport into the operating room, the opening of said container 4, by removing, in sequence, the plastic film 15, the means of coverage for evacuation 9 and the first means of coverage for sterilization 5. The support racks 18 containing the surgical instruments 2 are then extracted from the container 4 for the preparation of the operating table, having previously seen the graphic reproduction 3 for the execution of the surgery.

Step e) of the method object of the invention relates to the collection of the surgical instruments 2 used by the surgeon for the surgery, their re-insertion inside the container 4 and the closing of said container 4 with the apposition of the second means of coverage for evacuation 9, bound to the container 4 by, for instance, a restraining, interference or snap-lock means of fixture.

During said step e), before closing the container 4, a pre-treatment operation is carried out with biocidal solution for decontamination of the used surgical instruments 2, which are then sprinkled with substances which stop or slow the development of bacteria, such as, for instance, surfactant bacterostatic gel. Subsequently, said closed container 4 is put inside a collection trolley 7 for movement in safety toward the structure in which the operations of sanitizing, disinfection, maintenance, packaging and sterilization of the used surgical instruments 2, of the related rack 18 and of the related container 4 are carried out. Said collection trolley 7 can internally house a multiplicity of containers 4, each container 4 can contain a related set of surgical instruments 2 and related support rack 18.

Step f) of the method which is the subject of the present invention relates to the treatment of washing and decontamination of the used surgical instruments 2 and of the related container 4. According to a preferred, but not exclusive, embodiment of this method, said step f) can be preceded by a transport operation in a structure specifically arranged to receive said trolley and to carry out the operations of said step f). Said transport operation is suitably carried out by vehicles designed to receive at least one collection trolley 7 - each trolley containing at least one closed container 4 - and to guarantee its maximum stability during the transport.

Said collection trolley 7, containing said container 4, is transferred to a first acceptance area 101 of the structure 1 housing the environments in which the various steps of treatment of washing and decontamination of the surgical instruments 2, of the support racks 18, of the containers 4, and of the collection trolleys 7 are carried out.

Said structure 1 is specifically arranged for receiving said collection trolley 7, said containers 4 and said surgical instruments 2, since it is endowed with means for the independent regulation and control of the temperature, pressure and humidity, inside every environment.

Advantageously, said structure 1 has further environments 116, 117 and 118 for dressing of the employed personnel who carries out the requested operations, and for monitoring 115 the progress of the work for the various steps in each environment and for every container 4 entering, due to the presence of a means of automatic recognition of the sets of surgical instruments 2, of the containers 4 and of the support racks 18, such as, for instance, optical readers working on the passage of said set of surgical instruments, said containers and said racks, from one environment to another of the structure 1.

From the acceptance area 101 of said structure 1 the collection trolley 7 is moved toward the environment 105, passing through the environment 103, said environments 103 and 105 being temperature controlled and having pressure increasing from the environment 103 to the environment 105. Inside said environment 105, said containers 4 are withdrawn from said collection trolley 7 and transported toward the environment 106, where they are opened by the removal of the second means of air-tight coverage 9. Every open container 4 is emptied of the liquids resulting from the decontamination and together with its contents, comprising the set of surgical instruments 2 and the support racks 18, is subjected to a first pre-washing treatment. Said operation of pre-washing is performed preferably, but not exclusively, inside airtight containers provided with heating devices.

In the meantime, the collection trolley 7 is subjected to a treatment of sanitizing by passage through a treatment tunnel 104 in which, for instance, use is made of high pressure water-jets with detergents and disinfectants, and with a following biocide step with high-temperature steam, said trolley 7 subsequently being subjected to a drying cycle and finally transferred into the storage environment 113 for its reuse.

The surgical instruments 2, the containers 4 and the support racks 18, immediately after being subjected to the first pre-washing treatment, are transferred into environment 107 where they undergo a further manual washing treatment and decontamination, before being subjected to automatic washing operations. Said operations of manual washing and decontamination are carried out using solutions, such as, for instance, of a proteolytic enzyme type, and/or using a further step of treatment with ultrasounds. The preparation for the automatic washing is completed by the positioning of said surgical instruments 2, of said containers 4 and of said support racks 18 on special washing racks 8. At the end of step f) said surgical instruments 2 are checked and counted.

Said environment 107 is at a higher pressure than the environments 105 and 106.

Step g) of the method object of the present invention relates to the treatment of thermo-chemo-disinfection of the used surgical instruments 2, of the related container 4 and of the support racks 18. Said step is preferably, but not exclusively, carried out by automatic treatment in a washing device, such as, for instance, surgical instrument washer apparatuses 108, which are capable of submitting said instruments 2, said containers 4 and said support racks to specific cycles of treatment. At the end of the loading of said surgical instrument washer apparatuses by the positioning of the washing racks 8 in special baskets of every surgical instrument washer apparatus 108, said instruments 2, said containers 4 and said support racks 18 are subjected to washing cycles that can comprise, in particular, but not exclusively, one or more of the following operations:
washing
treatment with detergents
treatment with steam
lubricating treatment.

Said washing operation is carried out with substances, such as, for instance, tensioactive agents added to high pressure water jets.

Said treatment with detergents is carried out with substances, such as, for instance, enzymatic detergents.

Said lubricating treatment is carried out with substances, such as, for instance, emulsions of synthetic medical oils.

At the end of the cycle of automatic treatment, the surgical instruments 2, the containers 4 and the support racks 18 are subjected to drying while still inserted into the surgical instrument washer apparatus 108.

Step h) of the method object of the invention relates to the repackaging of the surgical instruments 2 supported on their related support racks 18 inside the related container 4. Said step h) is carried out inside the environment 109, which is subjected to a further control in order to reduce contaminants in the air and has a higher internal pressure than environment 107. Preferably, said surgical instrument washers are positioned in correspondence of the passage between the environment 107 and the environment 109, so as to constitute a further barrier for the progress of polluting substances from environment 107 to environment 109.

The operation of re-packaging is facilitated by the presence of the graphic reproduction 3 of the set of surgical instruments 2 subjected to treatment and from the presence of a bar code identification that allows to recall said graphic reproduction 3 as soon as the container 2 has passed through a mean of automatic recognition of the type constituted, for instance, by a optic reader.

The operation of repackaging the surgical instruments can be carried out, preferably, but not exclusively, on worktables of a plastic non-reflecting material to give a better visualization of the surgical instruments, and on work-surfaces having a certain plasticity such as to avoid damaging the same instruments 2 during their manipulation.

Inside the environment 109 said surgical instruments contents inside the related containers 4, preferably, but exclusively, can undergo a non-functionality check, and, where necessary, be replaced if worn or damaged.

Said damaged or worn instruments are, where necessary, replaced with new sanitized instruments stored near one or more storage environments 110.

Step i) of the method object of the invention relates to the application of the related means of coverage 5 on the container 4 containing the related set of surgical instruments 2 placed on the related racks 18 and following insertion of said closed containers 4 inside a suitable sterilization device, such as, for instance, an autoclave 111, with use of movement trolleys suitably constructed to house said containers 4 and to enter inside said autoclaves. Preferably, said autoclaves 111 are positioned in correspondence of the passage between environment 109 and environment 112, thus constituting, further barriers to the passage of polluting substances from environment 109 to environment 112. Said environment 112 it is kept at a higher air pressure than the environment 109.

The activation of the autoclaves 111 enables the sterilization of the containers 4, the surgical instruments 2 contained therein and of the related support racks 18 for said surgical instruments 2, due to the fact that the sterilization steam passes through the passages 12 made on the cover 5 of the container 4. The above sterilization operation is also carried out contemporarily on at least one test-tube to verify the functioning of the autoclave and can be preceded and followed by weighing the packaged container, using high precision weighing devices. Said weighing operation permits verification that, subsequent to the sterilization, there is no unsuitable condensate present inside the container 4.

At the end of the sterilization operation, the containers 4, the surgical instruments 2 and the related support racks 18 are taken from the autoclaves 111 to the side of the environment 112.

Step I) of the method which is the subject of the present invention is carried out inside the sterile environment 112 and relates to the application of a second means of coverage 9 that it is locked onto said container 4 by a means of fixture such as, for instance, restraining or interference or snap-lock means of fixture, and to the positioning, on the top of said second means of coverage 9, of the elements of identification of the container 4, such as, for instance, a graphic reproduction 3 of the set of surgical instruments 2 and a systems of identification with bar codes 17.

Step m) of the method object of the present invention relates to the sealing of the container in the sterile environment 112, through the application of a plastic film 15, preferably, but not exclusively, of the thermo-shrink type, that shrouds the closed container 4, and that, once heated, it is withdrawn guaranteeing a further airtight seal inside the closed container 4, the protection of the container and a further system to prevent any accidental opening of the container.

During said step m) the container 4, which comprises a first means of coverage 5, a second means of coverage 9, a means of graphic identification 3 and a plastic film 15, is moved to environment 114 and repositioned on the collection trolleys 7, previously sanitized and moved from environment 113 to environment 114.

Said environment 114 is kept at a higher pressure than environments 112and 113.

Step n) of the method object of the present invention relates to the transport of the collection trolleys 7 from the environment 114 to the environment 119, where they wait for their loading on the suitable vehicles and their transport to the hospital compartment, where the containers 4 are stored. The loading of said collection trolleys 7 on said vehicles happen in the loading zone 120 passing through the environment 102 of the structure 1.

It has been ascertained in practice that the method herein described, which is the subject matter of the present invention, achieves the purposes proposed.

In fact, said structure 1 is so arranged that the pressure of the air in every environment is lower than the pressure of the air in the following environment involving ever smaller degrees of contaminating substances, as one draws near to the sterilization step, in the environment 111, and of closing of the containers sterilized with further fitting of the plastic film 15 in the sterile environment 112.

Subsequently, advantageous is the layout of the various environments that, according to the method object of the present invention, guarantees optimal anti-bacteria protection of the surgical instruments due to the fact that from step d) to the step n) the operations carried out involve adjacent and connected environments through airtight passages or apparatuses with double openings.

It should be noticed that the operations that are carried out in environments 102, 103, 104, 105, 106, 107, 109, 112, 113, 114 and 119 present inside structure 1, are performed in advantageous way subsequently by personal wearing anti-contamination garments and footwear to protect themselves in the environments 106 and 107 and to protect the environments in 109, 112, 113, 114. Furthermore, all the passages that conduct from one environment to another one are made airtight to prevent the transfer of contaminating substances from one environment to another and falls of pressure.

Furthermore, the method object of the patent, associating for every type of surgery and, where necessary, for every surgeon a unique kit comprising container, surgical instruments and support racks, endowed with means of identification with bar codes or other methods, permits complete trackability to be guaranteed for every procedural set, step by step, and environment by environment, during every treatment and/or operation present in the step.

Said trackability is managed by operators present inside a further environment 115 present inside the structure 1 who, with the aid of computerized means, coordinate and organize the various steps of the method and can, where necessary, track down any container, surgical tool or rack 18 during any step.

## Claims

1. Container (4) for a set of sterilized surgical instruments (2) suitable for the execution of each particular surgery, comprising a sterilization cover (5) having passages (12) for the sterilization steam, second means of coverage (9) that is blocked onto said container for closing said container (4) and a support rack (18) on which said instruments (2) are supported, **characterized in that** it comprises a thermo-shrink film (15) that shrouds the container for airtight sealing of the closed container.

2. Container according to claim 1, **characterized in that** between said second means of coverage (4) and said plastic film (15) is placed a graphic reproduction (3) of the set of surgical instruments contained therein.

3. Method for packaging for a set of sterilized surgical instruments (2) by means of a container as claimed in claims from 1 to 3, comprising the following steps: repackaging of said surgical instruments inside said container; applying means of coverage (5) on the container for the sterilization of said container and said set of instruments; further it comprises the step of applying a second means of coverage (9) that is blocked onto said container, **characterized in that** said step of applying a second means of coverage is followed by the step of sealing the container through the application of a thermo-shrink film, that shrouds the closed container

4. Method according to claims 3, **characterized in that** said step of sealing said container through the application of a thermo-shrink film is preceded by the step of positioning on the top of said second means of coverage a graphic reproduction of the set of said surgical instruments.

## Patentansprüche

1. Behälter (4) für einen Satz von sterilisierten chirurgischen Instrumenten (2), geeignet zur Durchführung jeder besonderen Operation, umfassend eine Sterilisationsabdeckung (5) mit Durchgängen (12) für den Sterilisationsdampf, zweite auf dem Behälter arretierte Abdeckmittel (9) zum Verschließen des Behälters (4) und ein Tragegestell (18), auf welchem die Instrumente (2) gehalten sind, **dadurch gekennzeichnet, dass** der Behälter eine Thermo-Schrumpffolie (15) umfasst, welche den Behälter zum luftdichten Verschließen des geschlossenen Behälters umhüllt.

2. Behälter nach Anspruch 1, **dadurch gekennzeichnet, dass** zwischen dem zweiten Abdeckmittel (4) und der Kunststofffolie (15) eine graphische Wiedergabe (3) von dem darin enthaltenen Satz von chirurgischen Instrumenten angeordnet ist.

3. Verfahren zum Verpacken eines Satzes von sterilisierten chirurgischen Instrumenten (2) mittels eines in den Ansprüchen 1 bis 3 beanspruchten Behälters, umfassend die folgenden Schritte: Umpacken der chirurgischen Instrumente in den Behälter, Aufbringen von Abdeckmitteln (5) auf den Behälter zum Sterilisieren des Behälters und des Satzes von Instrumenten, weiterhin umfassend den Schritt des Aufbringens eines zweiten Abdeckmittels (9), welches an dem Behälter arretiert ist, **dadurch gekennzeichnet, dass** dem Schritt des Aufbringens eines zweiten Abdeckmittels der Schritt des Versiegelns des Behälters durch Aufbringen einer Thermo-Schrumpffolie folgt, welche den Behälter umhüllt.

4. Verfahren nach Anspruch 3, **dadurch gekennzeichnet, dass** dem Schritt des Versiegelns des Behälters durch Aufbringen einer Thermo-Schrumpffolie der Schritt des Positionierens einer graphischen Wiedergabe des Satzes der chirurgischen Instrumente auf dem zweiten Abdeckmittel vorausgeht.

## Revendications

1. Récipient (4) pour un ensemble d'instruments chirurgicaux stérilisés (2) approprié pour la réalisation de chaque chirurgie particulière, comprenant un couvercle de stérilisation (5) ayant des passages (12) pour la vapeur de stérilisation, des deuxièmes moyens de recouvrement (9) qui sont bloqués sur ledit récipient afin de fermer ledit récipient (4) et une grille de support (18) sur laquelle lesdits instruments (2) sont supportés, **caractérisé en ce qu'**il comprend un film thermorétractable (15) qui enveloppe le récipient pour l'étanchéité à l'air du récipient fermé.

2. Récipient selon la revendication 1, **caractérisé en ce qu'**entre lesdits deuxièmes moyens de recouvrement (4) et ledit film en plastique (15), on place une reproduction graphique (3) de l'ensemble d'instruments chirurgicaux contenus à l'intérieur de ce dernier.

3. Procédé pour emballer un ensemble d'instruments chirurgicaux stérilisés (2) au moyen d'un récipient selon l'une quelconque des revendications 1 à 3, comprenant les étapes suivantes consistant à : emballer à nouveau lesdits instruments chirurgicaux à l'intérieur dudit récipient ; appliquer les moyens de recouvrement (5) sur le récipient pour la stérilisation dudit récipient et dudit ensemble d'instruments ; il comprend en outre l'étape consistant à appliquer des deuxièmes moyens de recouvrement (9) qui sont bloqués sur ledit récipient, **caractérisé en ce que** ladite étape consistant à appliquer des deuxièmes moyens de recouvrement est suivie par l'étape consistant à réaliser l'étanchéité du récipient en appliquant un film thermorétractable, qui enveloppe le récipient fermé.

4. Procédé selon la revendication 3, **caractérisé en ce que** ladite étape consistant à réaliser l'étanchéité dudit récipient en appliquant un film thermorétractable est précédée par l'étape consistant à positionner sur la partie supérieure desdits deuxièmes moyens de recouvrement, une reproduction graphique de l'ensemble desdits instruments chirurgicaux.
